# EUROPEAN PATENT SPECIFICATION

(11) **EP 1 280 551 B1**
(45) Date of publication and mention of the grant of the patent: **10.01.2007**
(21) Application number: 01927532.0
(22) Date of filing: 25.04.2001
(51) Int. Cl.: A61K 39/12

(54) **VACCINES AGAINST HIV INFECTION**
IMPFSTOFFE GEGEN HIV
VACCINS CONTRE L'INFECTION VIH

(30) Priority: 27.04.2000 US 200011 P
(43) Date of publication of application: 05.02.2003
(60) Divisional application: 06076979.1
(73) Proprietor: Sanofi Pasteur Limited, Toronto, Ontario M2R 3T4 (CA)
(72) Inventor: ROVINSKI, Benjamin, Thornhill, Ontario L4J 5H6 (CA); TARTAGLIA, James, Schenectady, NY 12303 (US); CAO, Shi-Xian, Etobicoke, Ontario M9C 4X6 (CA); PERSSON, Roy, Toronto, Ontario M2M 4J4 (CA); KLEIN, Michel, H., Toronto, Ontario M4N 1B9 (CA)
(74) Representative: Bizley, Richard Edward
(86) International application number: PCT/CA2001/000577
(87) International publication number: WO 2001/082962

(56) References cited:
- WO-A-00/50604
- WO-A-91/05864
- WO-A-98/40501
- WO-A-99/31250
- CAVER T E ET AL: "A novel vaccine regimen utilizing DNA, vaccinia virus and protein immunizations for HIV-1 envelope presentation" VACCINE, BUTTERWORTH SCIENTIFIC. GUILDFORD, GB, vol. 17, no. 11-12, March 1999 (1999-03), pages 1567-1572, XP004158286 ISSN: 0264-410X
- EVANS T.G. ET AL.: "A Canarypox vaccine expressing multiple human immunodeficiency virus type 1 genes given alone or with rgp120 elicits broad and durable CD8+ cytotoxic T lymphocyte responses in seronegative volunteers." J. INFECT. DISEASES, vol. 180, 1999, pages 290-298, XP002185651

## Description

### FIELD OF THE INVENTION

The present invention relates to the field of immunology and, in particular, uses of materials for immunizing a host against infection with HIV-1.

### BACKGROUND OF THE INVENTION

Human immunodeficiency virus is a human retrovirus and is the etiological agent of acquired immunodeficiency syndrome (AIDS). It is estimated that more than 33 million people have been infected with HIV world-wide as of December 1999 (Ref 1- various references are referred to in parenthesis to more fully describe the state of the art to which this invention pertains. Full bibliographic information for each citation is found at the end of the specification, immediately preceding the claims. The disclosure of these references are hereby incorporated by reference into the present disclosure).

As the HIV epidemic continues to spread world wide, the need for an effective vaccine remains urgent. Efforts to develop such a vaccine have been hampered by several factors three of which are: (a) the extraordinary ability of the virus to mutate; (b) inability of most known specificities of anti-HIV antibodies to neutralise HIV primary isolates consistently; and (c) lack of understanding of the correlates of protective immunity to HIV infection. Over the last 10 years, several candidate HIV vaccines have been tested in primates for their immunoprotective abilities (Ref. 2). These studies suggest that both neutralising antibodies and cell-mediated immunity play a role in conferring sterilizing immunity and preventing progression towards disease (Ref 3, 4). While the correlates for immune protection against HIV-1 infection are currently unknown, an effective HIV vaccine should elicit both strong neutralising antibody and cytotoxic T lymphocyte (CTL) responses.

Envelope subunit vaccines have been shown to induce high titred humoral responses, but were inefficient in eliciting CTL responses (Ref 5). Live recombinant pox vectors have been shown to elicit very potent CTL responses, however these vectors were ineffective for generating a significant antibody response (Ref 6). In attempts to combine the two immunization types, several clinical trials involved a prime-boost strategy, consisting of initial viral vector immunization followed by boosts with recombinant HIV-1 envelope subunits (Ref 7, 8), have led to limited success with respect to CTL responses. Other vaccine approaches have used non-infectious, non-replicating, immunogenic virus-like particles (VLP) for immunising against HIV infection (Ref 9, 10). This type of immunogen has lead to the generation of neutralizing antibodies to a laboratory HIV-1 strain (Ref 10).

A prime-boost approach has been investigated using non-infectious VLPs to enhance HIV-specific CTL responses in mice primed with recombinant canarypox vector vCP205 encoding-HIV-1gp 120 (MN strain) (Ref 11). This study showed that VLPs could boost the CTL response to the canarypox vector. Another work describes the use of a DNA encoding env as a vaccine primer, followed by a booster with a vaccinia virus expressing env (Ref. 11 b). However, no material from primary isolates were used, and no neutralizing levels of antibodies were obtained.

Recently, a study showing the induction of neutralizing antibodies to a HIV-1 primary isolate in chimpanzees has been reported (Ref. 12). In this study, recombinant adenovirus expressing gp160 was used as the priming agent and recombinant gp120 was used to boost the monkeys.

There is still a need for vaccines and immunization regimes to induce both a strong CTL response as well as netralizing antibodies to HIV primary isolates

### SUMMARY OF THE INVENTION

In accordance with one aspect of the present invention, there is provided a vaccine for generating, in a host, particularly a human host, a virus neutralizing level of antibodies to a primary HIV-1 isolate. The administration regimen comprises at least one administration of a priming antigen to the host, wherein the priming antigen comprises a DNA molecule encoding an envelope glycoprotein of a primary isolate of HIV-1; resting the host for at least one specific resting period to provide for clonal expansion of an HIV-1 antigen specific population of precursor B-cells therein to provide a primed host; and at least one administration of a boosting antigen to the primed host to provide said neutralizing levels of antibodies, wherein the boosting antigen is an attenuated ALVAC viral vector expressing at least part of an envelope glycoprotein of a primary isolate of HIV-1. Also disclosed for comparative purpose is a vaccine in which the boosting antigen is a non-infections, non-replicating, immunogenic HIV-like particle (VLP) having at least part of the envelope glycoprotein of a primary isolate of HIV-1.

The primary HIV isolate will be an HIV-1 isolate including from the clade B HIV-1 clinical isolate HIV-1_{Bx08}, although any other primary HIV-1 isolate may be employed in the immunization procedures of the invention.

The DNA molecule encoding the envelope glycoprotein of a primary isolate of HIV-1 may be contained in a plasmid vector under the control of a heterologous promoter, preferably a cytomegalovirus promoter, for expression of the envelope glycoprotein in the host, which may be a human host.

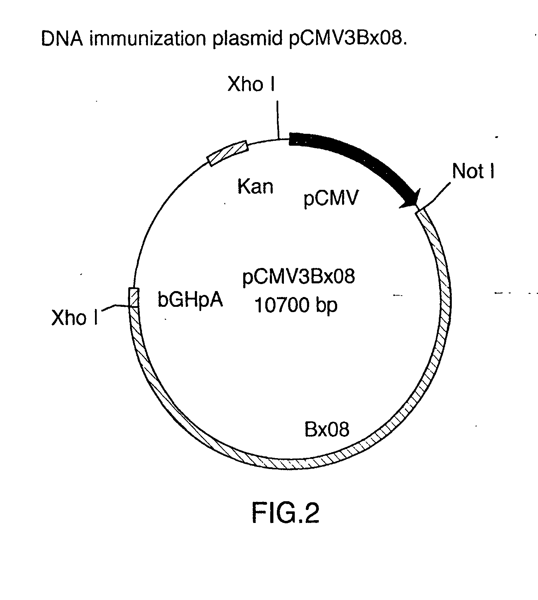

Preferably, the vector has the identifying characteristics of pCMV3Bx08 shown in Figure 2, such identifying characteristics being the nucleic acid segments and restriction sites identified in Figure 2.

A priming administration of antigen may be effected in a single or in multiple administrations of the priming antigen. In the latter case, the at least one specific resting period to permit clonal expression of HIV antigen-specific population precursor B-cells may be effected after each priming administration. The at least one specific resting period may be between about 2 and 12 about months.

Where the boosting antigen is a non-infectious, non-replicating, immunogenic HIV-like particle, such particle may comprise an assembly of:
(i) an *env* gene product,
(ii) a *pol* gene product, and
(iii) a *gag* gene product
with the particle being encoded by a modified HIV genome deficient in long terminal repeats (LTRs) and containing gag, pol and env in their natural genomic arrangement. Such particles and the manufacture thereof are described in US Patent No. 5,439,809, assigned to the assignee hereof and the disclosure of which is incorporated herein by reference. Such particles can include mutations in gag and pol to further reduce potential infectivity, as more fully described in United States Patent No. 6,080,408, assigned to the assignee hereof and the disclosure of which is incorporated herein by reference (WO 96/06177). The *env* gene is preferably that from primary isolate BX08. The gag gene and *pol* gene may be those from the same primary isolate or may be chosen from those of other HIV-1 isolates, which may be primary isolates.

The non-infectious, non-replicating, immunogenic HIV-like particle may be administered in conjunction with an adjuvant. Any suitable adjuvant may be used, such as QS21, DC-chol, RIBI or Alum. Such non-infectious, non-replicating, immunogenic HIV particle may be formed by expression from a suitable vector in mammalian cells.

There is described a vector comprising a modified HIV-genome deficient in long terminal repeats and a heterologous promoter operatively connected to said genome for expression of said genome in mammalian cells to produce the non-infectious, non-replicating and immunogenic particle, wherein at least the *env* gene of the modified HIV-genome is that from a primary isolate of HIV. The *gag* and *pol* genes of the modified HIV genome may be those from the same primary isolate or those from another isolate, which may be a primary isolate.

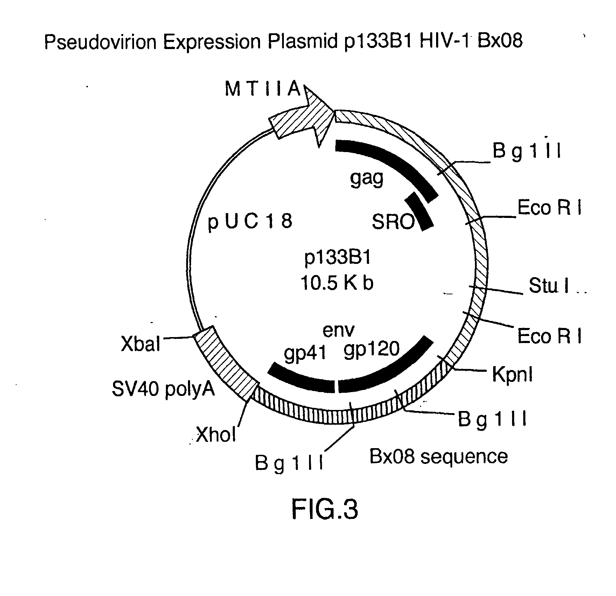

The vector preferably is a plasmid vector while the primary isolate preferably is BX08. The promoter may be the metallothionein promoter. The vector preferably has the identifying characteristics of plasmid p133B1 shown in Figure 3, such identifying characteristics being the nucleotide segments and restriction sites identified in Figure 3.

The boosting antigen of the invention is an attenuated viral vector, namely the attenuated canary poxvirus ALVAC. The attenuated viral vector may contain a modified HIV genome deficient in long terminal repeats (LTRs), wherein at least the env gene is that from primary isolate BX08. The *gag* and *pol* genes of the modified genome may be those from the same primary isolate or may be chosen from other HIV isolate.

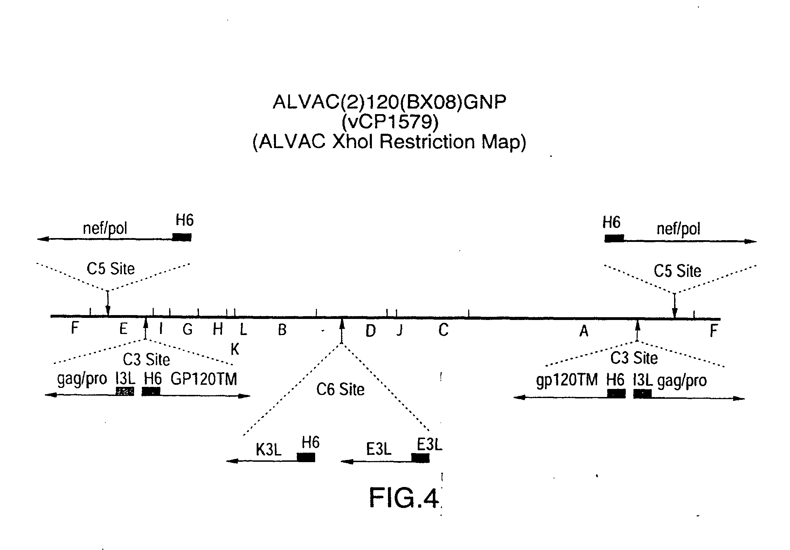

The attenuated canarypox virus-based vector ALVAC is a plaque-cloned derivative of the licensed canarypox vaccine, Kanapox, and is described in reference 19. The attenuated canary pox vector preferably has the identifying characteristics of vCP1579 shown in Figure 4, such identifying characteristics being the nucleic acid segments and restriction sites identified in Figure 4.

The at least one administration of a boosting antigen may be effected in a single administration or at least two administration of the boosting antigen.

The invention further includes compositions comprising the immunogens as provided herein and their use in the manufacture and formulation of immunogenic compositions including vaccines.

### BRIEF DESCRIPTION OF THE DRAWINGS

The present invention will be further understood from the following description with reference to the drawings, in which:
Figure 1 shows the details of the elements of plasmid pCMVgDtat⁻ vpr⁻Bx08.
Figure 2 shows the details of the elements of plasmid pCMV3Bx08.
Figure 3 shows the details of the elements of plasmid p133B1.
Figure 4 shows the details of the insertions into ALVAC (2) to provide vector vCP1579.
Figures 5A and 5B contain a representation in time-line form of the immunization regime used wherein the study groups are described in Table 1. The numbers below the lines refer to weeks.
Figure 6 shows the immunoreactivity to HIV-1 antigens of the serum diluted 1:100 from the macaques immunized with the various preparations as described in Table 1.
Figure 7 shows the immunoreactivity to HIV-1 antigens of the serum diluted 1:1000 from the macaques immunized with the various preparations as described in Table 1.
Figure 8 shows the details of the elements of pMPC6H6K3E3.
Figure 9 shows the details of the elements of pMPC5H6PN.
Figure 10 shows the details of the elements of pHIV76.
Figure 11 shows the nucleotide sequence (SEQ ID NO: 1) for the H6/HIV Pol/Nef epitope cassette in the ALVAC C5 site of vCP1579.
Figure 12 contains the nucleotide sequence of C6 region (coding strand SEQ ID NO: 16, complementary strand SEQ ID NO: 17, K3L amino acid sequence SEQ ID NO: 18, E3L amino acid sequence SEQ ID NO: 19).

### GENERAL DESCRIPTION OF INVENTION

As noted earlier, the present invention involves administration of HIV antigens to elicit virus-neutralizing levels of antibodies against a primary HIV isolate.

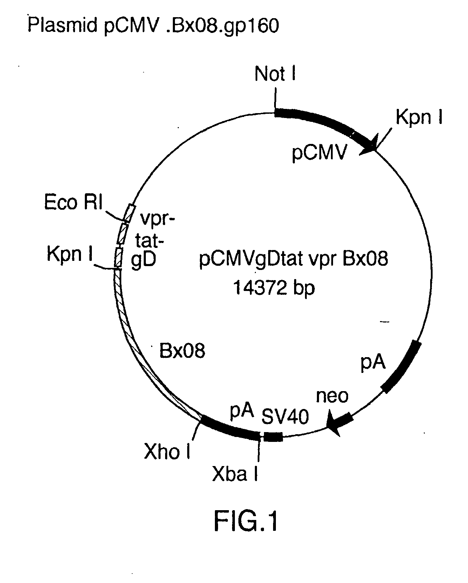

A DNA construct was prepared incorporating the env gene from the primary isolate Bx08 under the control of the cytomegalovirus promoter and the construct, pCMV3Bx08, is shown in Figure 2. The construct pCMV3Bx08 is derived from plasmid pCMVgDtat⁻vpr⁻Bx08 seen in Figure 1. The DNA construct pCMV3Bx08 was used in a priming immunization step to a host, macaque monkeys being the animal model chosen.

Following the priming immunization step, which may be effected in one or more administrations of the DNA construct, the host is allowed to rest to provide for clonal expression of an HIV antigen specific population of precursor B-cells therein to provide a primed host.

The boosting administration is effected either with a non-infectious, non-replicating, immunogenic HIV-like particle (VLP) (for comparative purposes) or an attenuated viral vector (according to the invention).

For this purpose, a VLP expression plasmid was constructed containing a modified HIV genome lacking long terminal repeats in which the *env* gene is derived from primary isolate BX08, wherein the modified HIV genome is under the control of a metallothionein promoter. The construct, p133B1, shown in Figure 3, was used to effect expression in mammalian cells of the non-infectious, non-replicating, immunogenic HIV-like particules, in which the *env* gene product is that from the primary isolate BX08.

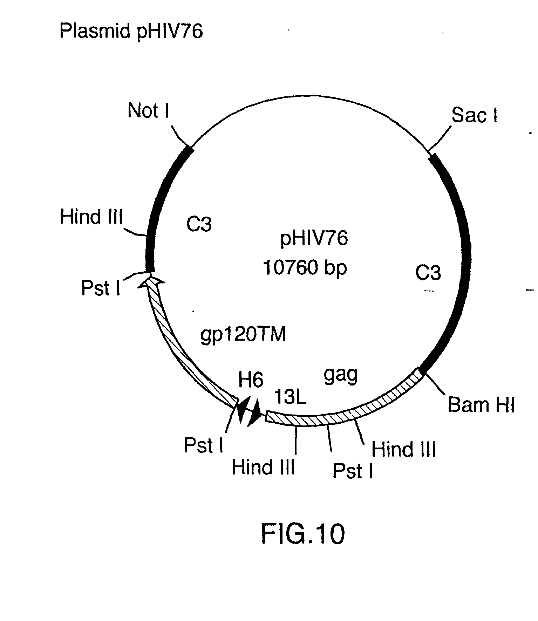

In the case of the attenuated virus vector, a recombinant attenuated canarypox virus vector was constructed to contain the *env* gene from primary isolate BX08. The viral vector vCP1579 (Figure 4) was prepared by a variety of manipulatious from plasmid pHIV76 (Figure 10), as shown described in detail below.

These products were utilized in a boosting administration to the primed macaques. The boosting administration may be effected in one or more immunizations. In a preferred aspect of the invention, the non-infectious, non-replicating immunogenic HIV-like particles may co-administered with the DNA construct in the priming administration and the DNA construct may be coadministered with the HIV-like particles in the boosting administration.

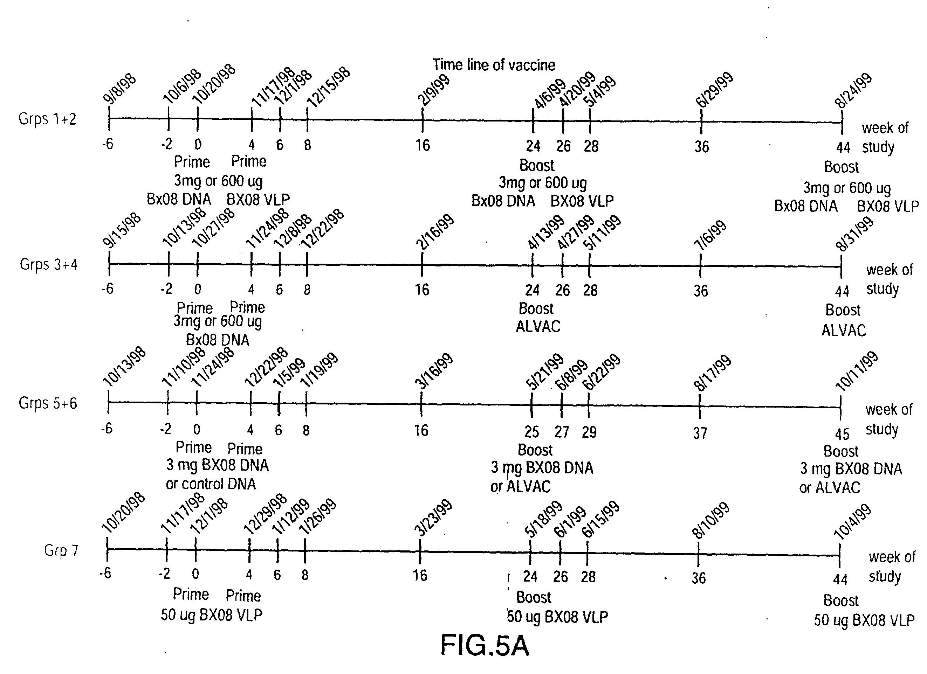

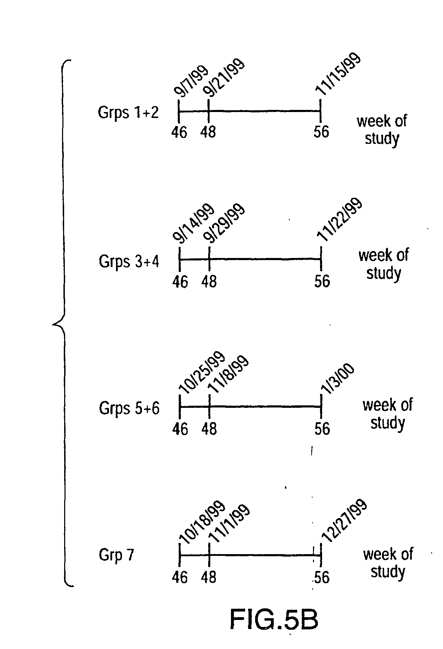

Immunizations were effected in accordance with the procedure described herein and the results obtained were compared with those obtained using other protocols according to the protocols set forth in Table 1. The immunization regimes used are shown as time lines in Figures 5A and 5B.

The results obtained following the various protocols showed that, in particular, a primary DNA vaccination in combination with a boost from either the VLP or the attenuated canarypox virus enhanced the levels of neutralizing antibodies, as indicated by the reduction of detectable p24 levels in cells infected with primary HIV isolates.

### Biological Deposits

Certain vectors that are described and referred to herein have been deposited with the American Type Culture Collection (ATCC) located at 10801 University Boulevard Manassas, Virginia 20110-2209, USA, pursuant the Budapest Treaty and prior to the filing of this application. Samples of the deposited vectors will become available to the public and all restrictions imposed on access to the deposits will be lifted upon grant of a patent based on this patent application. In addition, the deposit will be replaced if viable samples cannot be dispensed by the Depository. The invention described and claimed herein is not limited in scope by the biological materials deposited, since the deposited embodiment is intended only as an illustration of the invention. Any equivalent of similar vectors that contain nucleic acids which encode equivalent or similar antigens as described in this application are within the scope of the invention.

| Deposit Summary | | |
|---|---|---|
| Plasmid | ATCC | Deposit Date |
| pMT-HIV | 40912 | October 12, 1990 |
| pCMVgDtat⁻vpr⁻ | 209446 | November 11, 1997 |

### EXAMPLES

The above disclosure generally describes the present invention. A more complete understanding can be obtained by reference to the following specific Examples. These Examples are described solely for purposes of illustration and are not intended to limit the scope of the invention. Obvious Changes in form and substitution of equivalents are contemplated as circumstances may suggest or render expedient.

Example 1

This Example describes the construction of plasmid pCMV3BX08.

The plasmid, pCMV3BX08, contains sequence segments from various sources and the elements of construction are depicted in Figure 2.

The prokaryotic vector pBluescript SK (Stratagene) is the backbone of the plasmid pCMV3.BX08 and was modified by the replacement of the *Amp*^{R} with *Kan*^{R} gene and the deletion of the fl and the LacZ region. To achieve the desired modifications, the sequence between *Ahd*l (nucleotide 2,041) and *Sac*l (nucleotide 759) of pBluescript SK, which contains the *Amp*^{R}, fl origin and the LacZ, was deleted. A 1.2 kb *Pst*l fragment from the plasmid pUC-4K (Pharmacia) containing the *Kan*^{R} gene, was blunt end ligated to the *Ahd*l site of pBluescript SK in a counter-clockwise orientation relative to it's transcription. A 1.6 kb *Ssp*1/*Pst*1 DNA fragment containing the human cytomegalovirus immediate-early gene promotor, enhancer and intron A sequences (CMV) was ligated to the other end of the *Kan*^{R} gene so that the transcription from the CMV promoter proceeds in the clockwise orientation. A synthetic oligonucleotide segment containing translation initiation sequence and sequences encoding the human tissue plasminogen activator signal peptide (TPA) was used to link the CMV promotor and the sequences encoding the envelope gene of the primary isolate HIV-1_{BX08}.

The envelope gene from the HIV-1 primary isolate BX08 was isolated from the plasmid pCMVgDtat⁻vpr⁻Bx08 illustrated in Figure 1. The plasmid pCMVgDtat⁻vpr⁻Bx08 was derived from the deposited plasmid pCMVgDtat⁻vpr⁻, the construction of which is described in copending United States Patent Application No. 08/991,773 filed December 16, 1997, assigned to the assignee hereof and the disclosure of which is incorporated herein by reference, (WO 99/31250). The plasmid pCMVgDtat⁻vpr⁻Bx08 was derived by substituting the BX08 envelope sequence from clade B HIV-1 clinical isolate HIV-1_{BX08} for the modified HIV genome sequence present in pCMVgDtat⁻vpr⁻. Plasmid pCMVgDtat⁻vpr⁻Bx08 was restricted with the restriction enzyme *Xho* I and made blunt ended with Klenow treatment. A *Not* I partial digestion was then performed and the resulting 6.3 kb fragment containing the env gene was isolated. Plasmid pCMV3 (Invitrogen) was restricted with *Bam* HI and made blunt ended with Klenow treatment. The plasmid pCMV3 was then restricted with *Not* I and the resulting 4.4 kb fragment was isolated. The 6.3 and 4.4 kb fragments were ligated together to produce plasmid pCMV3BX08 (Figure 2).

The pCMV3BX08 construct was introduced into HB101 competent cells according to manufacturer's recommendations (GibcoBRL). Correct molecular clones were identified by restriction and sequencing analysis and their expression of envelope glycoprotein was examined in transient transfections followed by Western blot analysis.

All DNAs used for immunizations were prepared using EndoFree Plasmid Kit (Qiagen). For intramuscular immunizations either 3 mg or 600 µg of pCMVBX08, in 100 µl PBS was injected.

Proviral DNA for clade B HIV-1 clinical isolate HIV-1_{BX08} originated at Transgene (Strasbourg, France) and was isolated from genomic DNA of cells infected with the virus.

Example 2

This Example describes the construction of plasmid p133B1.

A Bx08 plasmid expression vector (p133B1, Figure 3) used to transfect the mammalian cells was engineered in several stages using pUC18 as the initial host plasmid. First, an 8.3-kbp fragment of HIV-1_{LAI} provirus encoding the gag, pol and env proteins was isolated. This fragment lacked the transcription regulatory elements and long terminal repeat elements from each end of the proviral genome to ensure the virus-like particles would be replication-incompetent. This fragment was linked to an inducible human type IIA metallothionein (MTIIA) promoter (Ref 13) and also to a Simian Virus 40 polyadenylation (polyA) addition/transcription termination sequence from plasmid pSV2dhfr (Ref 14). The modified fragment was then inserted into the pUC18 host vector. The resulting deposites expression construct, named pMT-HIV, was used to transfect into African green monkey kidney (Vero) and COS monkey kidney cells. The procedure for obtaining pMT-HIV is further described in the aforementioned US Patent No. 5,439,809. Both transfected cell lines produced non-replicating virus-like particles when induced with metal ions (Ref 15).

Two further modifications were made to the proviral DNA in pMT-HIV to provide additional safety features to protect human cells against recombination events with reverse-transcribed DNA:
1) inactivation of the RNA packaging sequences; and
2) deletion of a large section of the *pol* gene encoding reverse transcriptase and integrase.

To delete the first RNA packaging signal, part of the DNA corresponding to the untranslated leader sequence of the mRNA was replaced with synthetic DNA lacking a 25-bp motif corresponding to nucleotides 753-777 (the *psi* sequence). To inactivate the second RNA packaging signal, two adenosine residues within a *gag* gene zinc finger sequence were changed to thymidine residues. Each of these residue changes had the effect of replacing cysteine residues in a Cys-His array with a serine in the gene product.

The *pol* gene deletion was effected by replacing a 1.9-kbp fragment with synthetic DNA containing stop codons in all three reading frames. This prevented read-through translation of the residual integrase coding sequence on the 3' side of the deletion. The 1.9-kbp deletion in *pol* also eliminated the expression of reverse transcriptase and integrase enzymes. However, the deletion left intact the gene encoding the viral protease, which is both an immunogenic component of HIV-1 virus particles and allows the expression of particles with processed gag antigens closely resembling native virions (Ref 16). The protease also contains epitopes that are conserved across HIV-1 clades. The modifications described with respect to *gag* and *pol* genes are more fully described in the aforementioned United States Patent No. 6,080,408 (WO 96/06177).

Finally, the HIV-1_{LAI} *env* gene within pMT-HIV was replaced with that of HIV-1_{Bx08}. To effect this replacement, a 2440-bp fragment containing the *env* gene of Bx08 was amplified by polymerase chain reaction (PCR) from cells infected with this isolate. The PCR product was then used to replace the corresponding region present in pMT-HIV. However, the incoming fragment from HIV-1_{Bx08} was 125-bp shorter than the original BIV-1_{LAI} region owing to a deletion in the untranslated region between the env gene stop codon and the termination/polyA addition sequence. The resulting construct replaced all but eleven amino acid residues of the LAI envelope proteins gp120 and gp41. Of these eleven, only the first three differ between the LAI and Bx08 isolates, and these are all charge-conservative changes meaning the final expression vector (p133B1) encoded a nearly authentic HIV-1_{Bx08} env protein.

Example 3

This Example describes the production of HIV-like particles for comparative purposes.

African green monkey kidney (Vero) cells were recovered and cultivated in Dulbecco's modified Eagle medium (DMEM) containing 10% v/v fetal bovine serum (FBS), referred to below as Complete Medium. At passage 141, the cells were transfected with p133B1 using the calcium phosphate method when at approximately 30% confluence. The cells were shocked with glycerol 8 hours after transfection. For this step, six 10-cm dishes containing approximately 3.0 x 10⁶ cells each in 10.0 mL of Complete Medium were prepared. Each dish received 25.0 µg of expression vector and 2.0 µg of plasmid pSV2neo (Ref 17). The pSV2neo contains a selectable marker gene conferring resistance to the antibiotic geneticin (G418). Two days after transfection, the cells from each dish were recovered by trypsinization and replated into twenty-five fresh dishes in Complete Medium supplemented with 0.5 mg/mL of G418.

In total, 394 colonies were isolated from the dishes using cloning cylinders. Each colony was recovered by trypsinization and divided into two cluster dish wells, one of the wells per clone was induced after reaching 50% to 90% confluence. Prior to induction, the wells were treated by replacing all the medium with fresh Complete Medium containing 10.0 µM 5-azacytidine. After incubating for between 18 hours and 22 hours, the medium was removed and replaced with fresh DMEM containing 0.2% v/v FBS, 2.0 µM CdCl₂ and 200.0 µM ZnCl₂. The wells were incubated for a further 20 hours to 24 hours at which time samples of the medium were removed and tested by p24 ELISA.

The twenty highest-producing clones, based on the p24 titre, were chosen and cells from the corresponding uninduced wells were sub-cultured into one T-25 and one T-150 flask per clone. Both flasks were grown to confluence. The cells from the T-150 were recovered by trypsinization and cryopreserved at passage number 145. The cells from the T-25 were recovered by trypsinization every 3 days to 4 days and maintained up to passage 153. The cells were induced as above and samples retested by p24 ELISA at two different passages prior to passage 153.

The two highest p24 producers were chosen and were recovered by trypsinization every 3 days to 4 days up to passage 163. Samples from the clones were tested by p24 and gp120 ELISA from passage 158 and by p24 ELISA at passage 163, to assess clonal stability. The most suitable of these two cell lines, named 148 to 391, was chosen for further sub-cloning. The clone nomenclature defines the experiment number for this procedure, which was 148, and the number of the clone, which was number 391 of the original 394 isolated.

The vero cells were grown for approximately 100 h to 103 h and the medium was then replaced with growth medium containing 5-azacytidine. The bottles were then incubated for a further 20 h to 22 h, at which time the medium was replaced with serum-free medium containing CdCl₂ and ZnCl₂. The bottles were then incubated for 29 h to 31 h, at which time the medium was harvested, pooled and stored at 2°C to 8°C prior to purification.

The next day after harvesting, the solution was clarified, concentrated and diafiltered against phosphate buffer. The concentrate was passed through a ceramic hydroxyapatite (type I) column and the run-through was collected. The run-through from two successive sublots was pooled together and pumped onto a sucrose density gradient in a continuous zonal ultracentrifuge rotor. Pseudovirion-containing fractions were collected and pooled. The pooled pseudovirion fractions were diafiltered against PBS containing 2.5% sucrose to reduce the sucrose content, concentrated and diafiltered again. The material was sterile filtered using a 0.2 µm filter. At this stage the materials was designated as a purified sub-lot and were stored at 2 to 8°C.

The adjuvants were prepared separately and filter sterilized before filling in single dose vials. QS21 was stored at -20°C.

Example 4

This Example describes the production of recombinant pox virus vCP1579.

Recombinant pox virus vCP1579 (Figure 4) contains the HIV-1 gag and protease genes derived from the HIV-1 IIIB isolate, the gp120 envelope sequences derived from the HIV-1 Bx08 isolate, and sequences encoding a polypeptide encompassing the known human CTL epitopes from HIV-1 Nef and Pol.

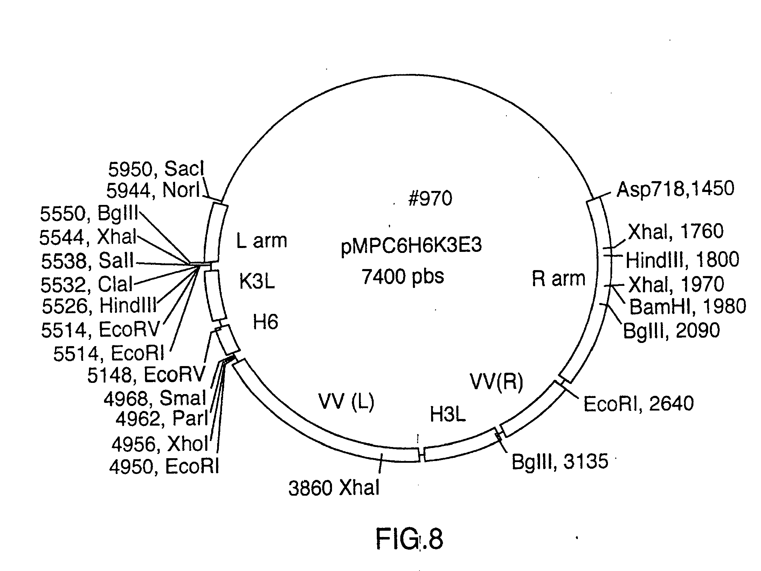

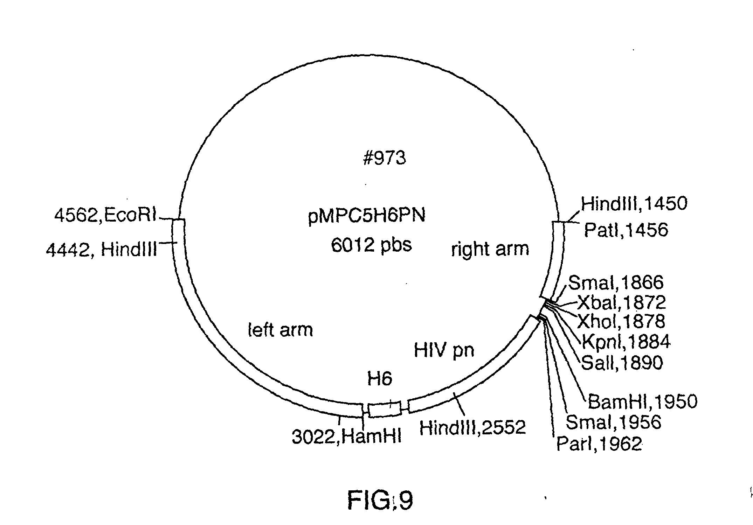

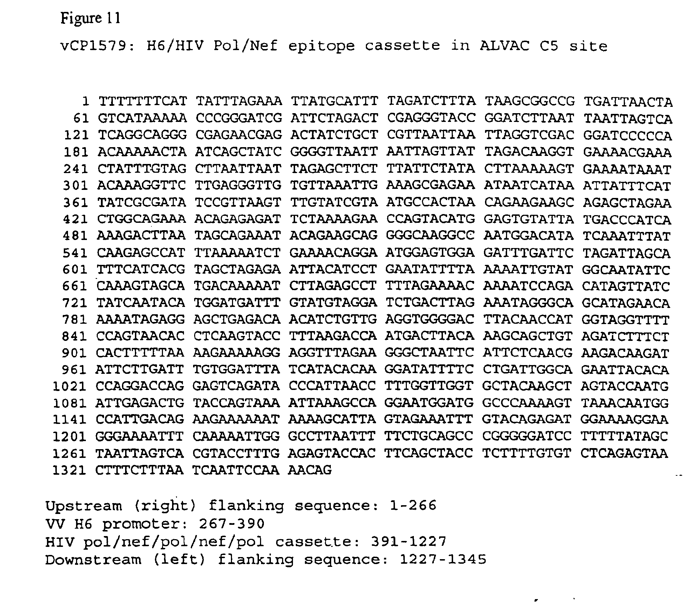

Recombinant vCP1579 (Figure 4) was generated by insertion of the vector modifying sequences from pMPC6H6K3E3 (Figure 8) encoding E3L and K3L into the C6 site of recombinant vCP1566 (Figure 4). Recombinant vCP1566 was generated by insertion of an expression cassette encoding a synthetic polypeptide containing Pol CTL epitopes and Nef CTL epitopes (Figure 11) and plasmid pMPC5H6PN (Figure 9) into vCP1453 at the insertion site known as C5. Recombinant vCP1453 was generated by co-insertion of genes encoding HIV-1 env and gag/protease gene products, plasmid pHIV76 (Figure 10), into the ALVAC genome at the insertion site known as C3.

The construction of recombinant pox vectors containing the E3L and K3L genes has been described in United States patent 6,004,777 issued Dec 21, 1999 to Tartaglia et al. and the recombinant pox vectors describing the insertion of HIV genes has been described in United States patent 5,766,598 issued June 16 1998 to Paoletti et al.

The locus designated C3 was used for the insertion of the HIV-1 *env* and *gag* gene sequences into the ALVAC(2) vector, and the locus designated as C5 was the insertion site for the sequences encoding the HIV-1 Nef and Pol CTL epitopes. By virtue of the C3 and C5 loci existing within the extensive inverted terminal repetitions (ITRs) of the virus genome (approximately 41 kbp), insertion into these loci results in the occurrence of two copies of the inserted HIV-1 sequences.

Briefly, expression cassette pHIV76 (Figure 10) was engineered in the following manner. Plasmid p133B1 (Figure 3) containing the HIV-1Bx08 gp 160 gene was used as the starting plasmid. The 3'-end of the H6 promoter was cloned upstream of the gp160 gene and three poxvirus early transcription termination signal sequences (T₅NT) were modified. This was accomplished by cloning a 2,600 bp *Bam*HI-digested PCR fragment, containing the 3'-end of the H6 promoter and the T₅NT-modified HIV-1 (BX08) gp160 gene, into the *Bam*HI site of pBS-SK. This PCR fragment was generated from four overlapping PCR fragments (a 570 bp fragment, a 140 bp fragment, a 500 bp fragment and a 1,450 bp fragment) and the oligonucleotides, RW835 (5'-ATCATCATCGGATCC CGGGGTCGCGATATCCGTTAAGTTTGTATCGTAATGAAAGTGAAGGAC C-3' - SEQ ID NO: 2) and RW836 (5'-ATCATCATCGGATCCCGGGGTT ATAGCAAAGCCCTTTC-3' - SEQ ID NO: 3). The 570 bp PCR fragment, containing the 3'-end of the H6 promoter and the 5'-end of the gp160 gene, was generated from the plasmid, p133B1, with the oligonucleotides, RW835 (5'-ATC ATCATCGGATCCCGGGGTCGCGATATCCGTTAAGTTTGTATCGTAATG AAAGTGAAGGAGACC-3') and RW868 (5'-ATCAAGACTATAGAAGA GTGCATATTCTCTCTTCATC-3'). The 140 bp PCR fragment, containing an interior portion of the gp160 gene, was generated from plasmid p133-B1 with the oligonucleotides, RW864 (5'-GCACTCTTCTATAGTCTTGATATAGTAC-3' - SEQ ID NO: 4) and RW865 (5'-AGCCGGGGCGCAGAAATGTATG GGAATTGGCAC-3' - SEQ ID NO: 5). The 500 bp PCR fragment, containing an interior portion of the gp160 gene, was generated from 133-3 with the oligonucleotides, RW866 (5'-ATACATTTCTGCGCCCCGGCTGGT TTTGCGATTC-3' - SEQ ID NO: 6) and RW867 (5'-GAAGAATTC CCCTCCACAATTAAAAC-3' - SEQ ID NO: 7). The 1,450 bp PCR fragment, containing the 3'-end of the gp160 gene, was generated from p133-B1 with the oligonucleotides, RW869 (5'-TGTGGAGGGGAATTCTTCTACTGTAATAC AACACAAC-3' - SEQ ID NO: 8) and RW836 (5'-ATCATCATCGGAT CCCGGGGTTATAGCAAAGCCCTTTC-3' - SEQ ID NO: 9). The 3'-end of the 570 bp PCR fragment overlaps the 5'-end of the 140 bp PCR fragment. The 3'-end of the 140 bp PCR fragment overlaps the 5'-end of the 500 bp PCR fragment. The 3'-end of the 500 bp PCR fragment overlaps the 5'-end of the 1450 bp PCR fragment. The plasmid generated by this manipulation is called pRW997.

The sequence encoding gp41 was then replaced with the sequence encoding the gp 160 transmembrane (TM) region. This modification was accomplished by cloning a 200 bp *Mfe*I-*Hin*dIII-digested PCR fragment, containing the 3'-end of the gp 120 gene and the TM sequence, into the 4,400 bp *Mfe*I*-Hin*dIII fragment of pRW997. This PCR fragment was generated from two overlapping PCR fragments (a 170 bp fragment and a 125 bp fragment) with the oligonucleotides, HIVP97 (5'-TAGTGGGAAAGAGATCTTCAGACC-3' - SEQ ID NO: 10) and HIVP101 (5'-TTTTAAGCTTTTATCCCTGCCTAACT CTATTCAC TAT-3' - SEQ ID NO: 11). The 170 bp PCR fragment was generated from pRW997 with the oligonucleotides, HIVP97 (5'-TAGTGGGAAAGAGATCTTCAGACC-3' - SEQ ID NO: 12) and HIVP100 (5'-CCTCCTACTATCATTATGAATATTCTTTTTTCTCTCTGCACCACTCT-3' -SEQ ID NO: 13). The 125 bp PCR fragment was generated from pRW997 with the oligonucleotides, HIVP99 (5'-AGAGTGGTGCAGAGAGAAAAA AGAATATTCATAATGATAGTAGGAGGC-3' - SEQ ID NO: 14) and HIVP101 (5'-TTTTAAGCTTTTA TCCCTGCCTAACTCTATTCACTAT-3' - SEQ ID NO: 15). The plasmid generated by this manipulation is called pHIV71.

The H6-promoted gp120+TM gene was then cloned between C3 flanking arms, into a plasmid containing the I3L-promoted HIV1 gag/(pro) gene. This modification was accomplished by cloning the 1,600 bp *Nru*I-*Xho*I fragment of pHIV71, containing the H6-promoted gp120+TM gene, into the 8,200 bp *Nru*I-*Xho*I fragment of pHIV63 . The plasmid generated by this manipulation is called pHIV76 (Figure 10). Plasmid pHIV76 was used in *in vivo* recombination experiments with ALVAC (CPpp) as rescue virus to yield vCP1453.

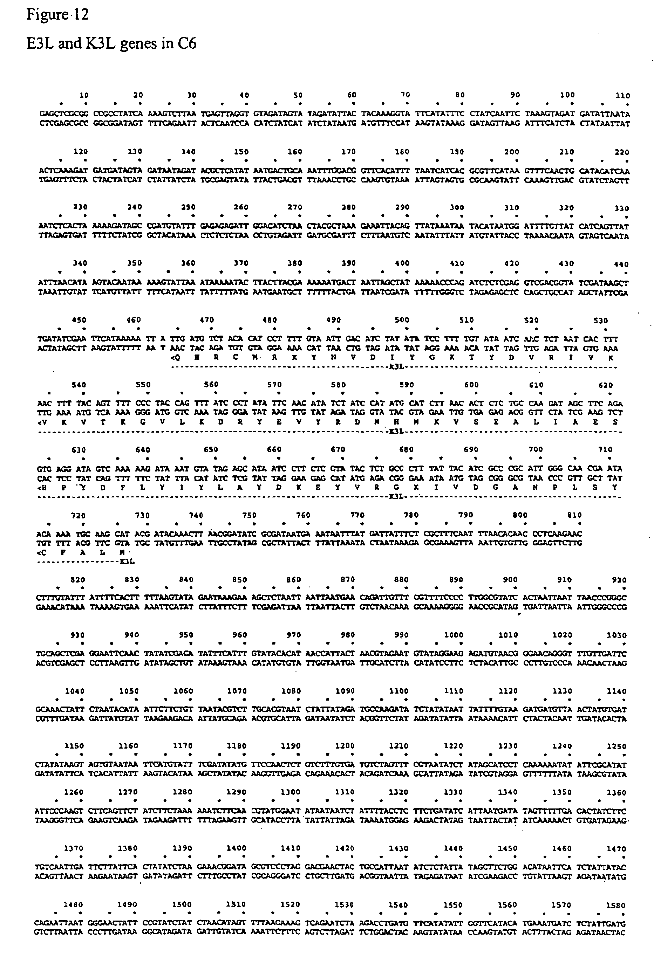

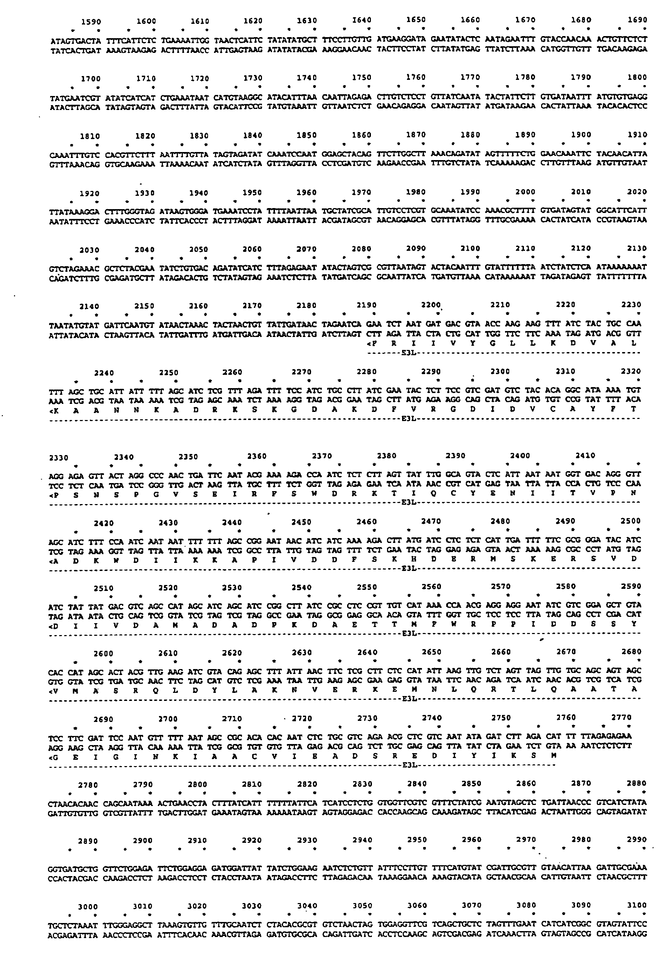

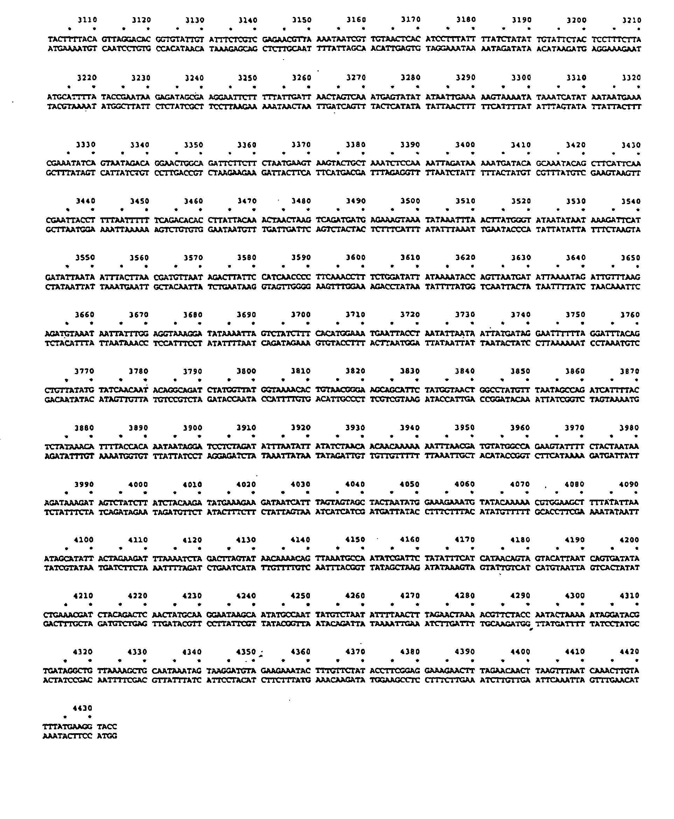

The sequence of the nef/pol regions is shown in Figure 12 and the E3L and K3L sequences are shown in Figure 13. To generate ALVAC(2)120(BX08)GNP (vCP1579), expression cassettes consisting of the promoter/HIV-1 gene combinations were subcloned into an ALVAC donor plasmid, which were then used to insert the expression cassettes into defined sites in the ALVAC genome by *in vitro* recombination as previously described (Ref 20).

Example 5

This Example describes the results of immunization regimes.

Groups of four animals (macaques) each were randomly assigned to seven vaccine groups as illustrated in Table 1. In this Table, "BX08 DNA" refers to pCMV3BX08, prepared as described in Example 1, "BX08 VLP" refers to the pseudovirions produced by expression vector p133B1 in Vero cells, as described in Example 3, and "ALVAC(2) BX08" refers vCP1579, prepared as described in Example 4. Reference (pre-bleed) sera were sampled at -6 and -2 weeks pre-vaccination. Primary immunizations with the various vaccines were given on weeks 0 and 4 with boosts on weeks 24 and 44 (Figures 5A, 5B). The vaccines were immunized intramuscularly into one quadricep of each macaque monkey.

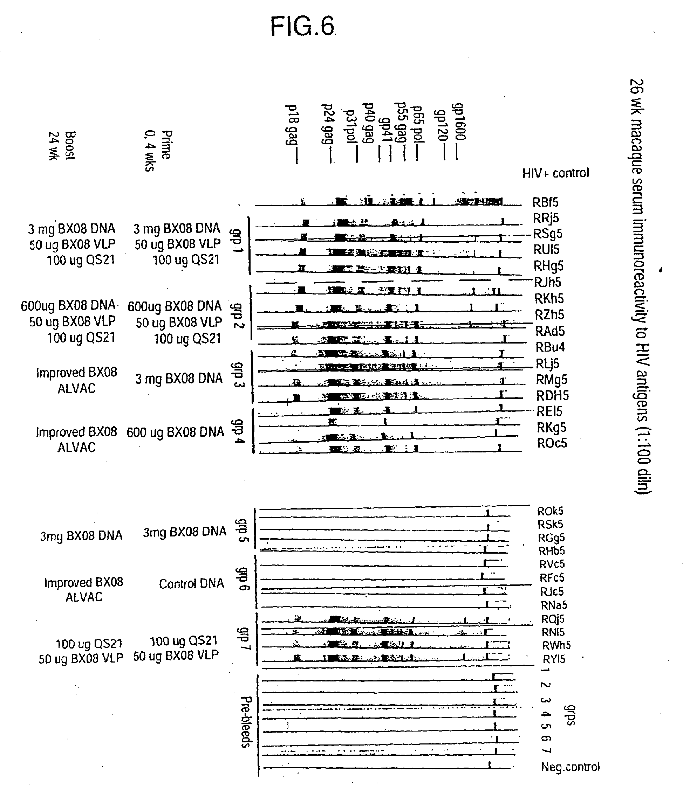

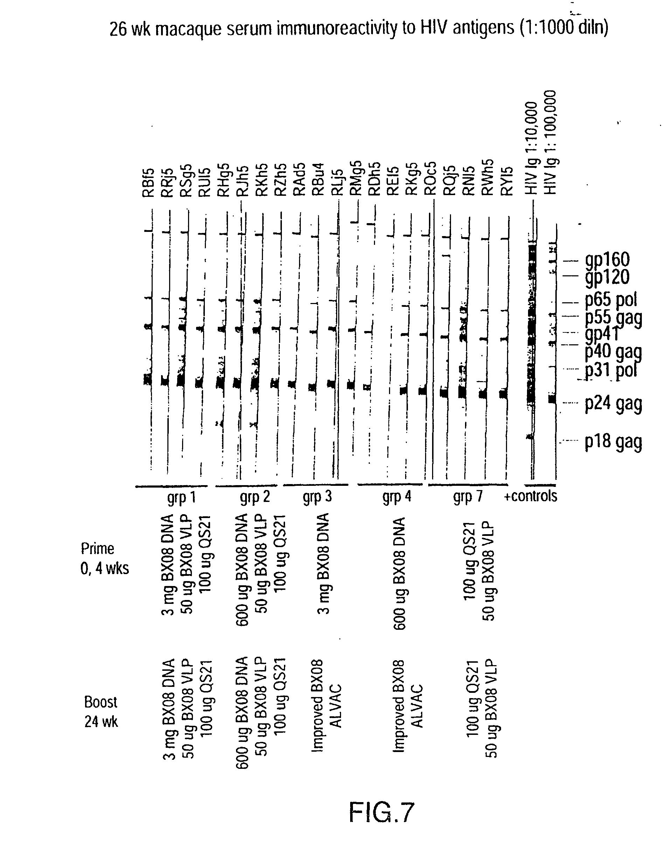

Sera were prepared from whole-blood using SST collection tubes and analyzed using commercially available HIV-1 western blots. Groups 1, 2 and 7 showed low levels of anti-Env antibodies after the first boost (Figures 6 and 7). Based on ELISA values, the anti-env antibody levels were below 1 µg/ml of specific IgG. High levels of anti-gag antibodies were detected in groups 1, 2, 3, 4, and 7 (Figures 6 and 7). No HIV-1 specific antibodies were detected in groups 5 and 6 (Figure 6).

The ability of the antibodies raised in the immunized monkeys to neutralize HIV-1BX08 virus in human PBMC was assayed based on the reduction of p24 levels.

The neutralization assay was performed essentially as described in reference 18. Briefly, serum dilutions were mixed with HIV-1 BX08 and the mixtures incubated for 1 hour, then added to susceptible human PBMC cells. Titres were recorded as the dilution of serum at which p24 was reduced by 80%. Serum samples were assayed at 1:2, 1:8 and 1:32 dilution on the virus (1:6, 1:24 and 1:26 dilutions after the addition of cells). p24 levels were evaluated by p24-specific ELISA assay.

DNA vaccination on its own, group 5, and ALVAC on its own, group 6, had no monkeys showing reduction of p24 levels greater than 80%. The low DNA (600 ug) plus ALVAC, group 4, also showed no monkeys with greater than 80% reduction of p24 titres. VLP plus DNA, either high or low dose (group 1 and 2) showed enhanced reduction of p24 levels compared to VLPs alone, group 7. High dose DNA, group 3, in combination with ALVAC enhanced the ability to elicit p24 or virus neutralising antibodies over the low dose, group 4 or ALVAC alone, group 6. These results indicate that DNA vaccination in combination with VLPs or ALVAC enhanced the levels of virus neutralising antibodies as indicated by the reduction of p24 levels in the sera of the immunized monkeys.

The percentage reduction of p24 is calculated relative to the amount of p24 produced in the presence of the corresponding dilution of week 2 samples.

### SUMMARY OF DISCLOSURE

In summary of this disclosure, the present invention provides novel immunogenic compositions for generating virus neutralizing levels of antibodies to a primary HIV isolate, to be used according to specific immunization procedures. Vectors utilized therein and for the generation of components for use in said composition are also disclosed. Obvious modifications are possible within the scope of this invention.

**Table 1 Study Design**

| **Group number** | **Treatment - Week 0, 4** | **Treatment - Week 24,44** |
|---|---|---|
| | 3 mg BX08 DNA | 3 mg BX08 DNA |
| 1 | 50 µg BX08 VLP | 50 µg BX08 VLP |
| | 100 µg QS21 | 100 µg QS21 |
| | 600 µg BX08 DNA | 600 µg BX08 DNA |
| 2 | 50 µg BX08 VLP | 50 µg BX08 VLP |
| | 100 µg QS21 | 100 µg QS21 |
| 3 | 3 mg BX08 DNA | ALVAC(2) BX08 (1x10⁸ pfu) |
| 4 | 600 µg BX08 DNA | ALVAC(2) BX08 (1x10⁸ pfu) |
| 5 | 3 mg BX08 DNA | 3 mg BX08 DNA |
| 6 | Control DNA | ALVAC(2) BX08 (1x10⁸ pfu) |
| 7 | 50 µg BX08 VLP | 50 µg BX08 VLP |
| | 100 µg QS21 | 100 µg QS21 |

**Table 2 Number of Monkeys showing > 80% reduction of p24 titre.**

| **Group number** | **Week 26 Bleed** | **Week 44 Bleed** |
|---|---|---|
| 1 | 3/4 | ¾ |
| 2 | 3/4 | 4/4 |
| 3 | 2/4 | 2/4 |
| 4 | 0/4 | 0/4 |
| 5 | 0/4 | 0/4 |
| 6 | 0/4 | 0/4 |
| 7 | 2/4 | 3/4 |

### REFERENCES

1. UNAIDS, WHO. AIDS epidemic update: December 1999. Geneva: World Health Organisation; 1999.
2. Heyward et al 1998. HIV vaccine development and evaluation: realistic expectations. AIDS Res Hum Retrivir 14:S205-S210.
3. Haigwood NL and Zolla-Pazner S. 1998. Humoral immunity to HIV, SIV and SHIV. AIDS 12: S121-S132.
4. Johnson et al . 1998. Cellular immune responses to HIV-1. AIDS 12:S113-120.
5. Keefer, M. et al 1996. AIDS Res Hum Retroviruses. 12:683-693.
6. Cox W. et al 1993. Virology 195:845-850.
7. Graham BS, Keefer MC, McElrath MJ, Gorse GJ, Schwartz DH, Weinhold K, Matthews TJ, Esterlitz JR, Sinangil F, Fast PE . 1996. *Ann Intern Med* Aug 15;125(4):270-9.
8. Pincus SH, Messer KG, Cole R, Ireland R, VanCott TC, Pinter A, Schwartz DH, Graham BS, Gorse GJ. 1997. *J Immunol* 1997 Apr 1;158(7):3511-20.
9. Fang ZY et al. 1999. J Infect Dis. 180(4):1122-32.
10. Rovinski B et al. 1995. AIDS Res Hum Retroviruses. 11:1187-1195.
11. Arp J. et al. 1999. Viral Immunolgy 12(4):281-296.
11b. Caver T et al., 1999. Vaccine March 1999; 17:1567-1572
12. Zolka-Pazner et al, J. Virology, vol. 72:1052-1059, 1998.
13. Karin M and Richards RI. Human metallothionein genes - primary structure of the metallothionein-II gene and a related processed gene. Nature 1982;299:797-802.
14. Sambrook J *et al*. Molecular Cloning A Laboratory Manual, Second Ed.: Cold Spring Harbour Laboratory Press. 1989.
15. Haynes JR, Cao SX, Rovinski B, Sia C, James O, Dekaban GA, Klein MH. Production of immunogenic HIV-1 viruslike particles in stably engineered monkey cell lines. AIDS Res Hum Retroviruses 1991;7:17-27.
16. Persson R, Cao S, Cates G, Yao F, Klein M, Rovinski B. Modifications of HIV-1 Retrovirus-like Particles to Enhance Safety and Immunogenicity. Biologicals 1998, 26(4): 255-265.
17. Southern PJ and Berg P. Transformation of mammalian cells to antibiotic resistance with a bacterial gene under control of the SV40 early region promoter. J Molec Appl Genet 1982;1: 327-341.
18. Graham, B.S. et al, 1993, J. Infection Des. 167:533-537.
19. Tartaglia, J. et al., 1992, Virology, 188:219-232.
20. Piccini, A. et al., 1987, Methods in Enzymology 153:545-563.

## Claims

1. The use of (i) a DNA molecule encoding an envelope glycoprotein of a primary isolate of HIV-1 and (ii) an attenuated ALVAC virus expressing at least an envelope glycoprotein of a primary isolate of HIV-1 in the manufacture of a medicament for generating a virus neutralizing level of antibodies to the primary HIV-1 in a host by through a regimen comprising:
(a) at least one administration of the DNA molecule encoding an envelope glycoprotein of a primary isolate of HIV-1 as a priming antigen,
(b) resting the host to allow clonal expansion of the precursor B-cells specific for the priming antigen,
(c) at least one administration to the primed host of the attenuated ALVAC virus expressing at least an envelope glycoprotein of a primary isolate of HIV-1 as a boosting antigen to provide the neutralizing levels of antibodies.

2. The use claimed in claim 1, wherein the primary isolate of HIV-1 is BX08.

3. The use claimed in claims 1 or 2, wherein the host rests for a period of 2 to 12 months.

4. The use claimed in any of claims 1 to 3, wherein the boosting antigen is administered at least twice.

5. The use claimed in any of claims 1 to 4, wherein said DNA molecule is contained in a plasmid vector under the control of a heterologous promoter for expression of the envelope glycoprotein in the host.

6. The use claimed in claim 5, wherein the heterologous promoter is the cytomegalovirus promoter.

7. The use claimed in any of claims 1 to 6, wherein the vector is pCMV3Bx08 shown in Figure 2.

8. The use claimed in any one of claims 1 to 7, wherein the priming antigen is administered at least twice, and wherein there host rests to allow clonal expansion of the precursor B-cell population specific for the priming antigen after each administration of said priming administration.

9. The use claimed in any one of claims 1 to 8, wherein the attenuated avipoxvirus vector contains a modified HIV-genome deficient in long terminal repeats, wherein at least the *env* gene is that from primary isolate BX08.

10. The use claimed in claim 9, wherein the attenuated ALVAC virus is vCP1579.

## Patentansprüche

1. Verwendung (i) eines DNA-Moleküls, das ein Hüllglycoprotein eines primären HIV-1-Isolats codiert, und (ii) eines attenuierten ALVAC-Virus, das wenigstens ein Hüllglycoprotein eines primären HIV-1-Isolats exprimiert, zur Herstellung eines Medikaments für die Erzeugung einer virusneutralisierenden Konzentration an Antikörpern gegen primäres HIV-1 in einem Wirt mittels eines Therapieschemas, umfassend:
(a) wenigstens eine Verabreichung des DNA-Moleküls, welches ein Hüllglycoprotein eines primären HIV-1-Isolats als Priming-Antigen codiert,
(b) Ruhenlassen des Wirts, um eine klonale Expansion der für das Priming-Antigen spezifischen Vorläufer-B-Zellen zu ermöglichen,
(c) wenigstens eine Verabreichung des attenuierten ALVAC-Virus, das wenigstens ein Hüllglycoprotein eines primären HIV-1-Isolats exprimiert, als Boosting-Antigen an den geprimten Wirt, um die neutralisierenden Konzentrationen an Antikörpern bereitzustellen.

2. Verwendung nach Anspruch 1, wobei das primäre HIV-1-Isolat BX08 ist.

3. Verwendung nach Anspruch 1 oder 2, wobei der Wirt für eine Zeitdauer von 2 bis 12 Monaten ruht.

4. Verwendung nach einem der Ansprüche 1 bis 3, wobei das Boosting-Antigen wenigstens zweimal verabreicht wird.

5. Verwendung nach einem der Ansprüche 1 bis 4, wobei das DNA-Molekül in einem Plasmidvektor unter der Kontrolle eines heterologen Promotors zur Expression des Hüllglycoproteins in dem Wirt enthalten ist.

6. Verwendung nach Anspruch 5, wobei der heterologe Promotor der Cytomegalovirus-Promotor ist.

7. Verwendung nach einem der Ansprüche 1 bis 6, wobei der Vektor der in Figur 2 gezeigte Vektor pCMV3Bx08 ist.

8. Verwendung nach einem der Ansprüche 1 bis 7, wobei das Priming-Antigen wenigstens zweimal verabreicht wird und wobei der Wirt nach jeder Verabreichung der Priming-Verabreichung ruht, um eine klonale Expansion der für das Priming-Antigen spezifischen Vorläufer-B-Zellpopulation zu ermöglichen.

9. Verwendung nach einem der Ansprüche 1 bis 8, wobei der attenuierte Avipoxvirusvektor ein modifiziertes HIV-Genom enthält, das in den langen terminalen Sequenzwiederholungen defizient ist, wobei wenigstens das *env*-Gen dasjenige des primären Isolats Bx08 ist.

10. Verwendung nach Anspruch 9, wobei das attenuierte ALVAC-Virus vCP1579 ist.

## Revendications

1. Utilisation (i) d'une molécule d'ADN codant pour une glycoprotéine d'enveloppe d'un isolat primaire de VIH-1 et (ii) d'un virus ALVAC atténué exprimant au moins une glycoprotéine de l'enveloppe d'un isolat primaire de VIH-1 dans la fabrication d'un médicament pour la génération d'anticorps dirigés contre le VIH-1 primaire chez un hôte, à un niveau neutralisant le virus, par un protocole comprenant :
(a) au moins une administration de la molécule d'ADN codant pour une glycoprotéine d'enveloppe d'un isolat primaire de VIH-1 en tant qu'antigène de primo-immunisation,
(b) une période de repos pour que l'expansion clonale des lymphocytes B précurseurs spécifiques de l'antigène de primo-immunisation puisse s'effectuer chez l'hôte,
(c) au moins une administration, à l'hôte primo-immunisé, du virus ALVAC atténué exprimant au moins une glycoprotéine de l'enveloppe d'un isolat primaire de VIH-1 comme antigène de rappel pour obtenir des niveaux d'anticorps neutralisants.

2. Utilisation selon la revendication 1, dans laquelle l'isolat primaire du VIH-1 est BX08.

3. Utilisation selon la revendication 1 ou 2, dans laquelle la période de repos de l'hôte est de 2 à 12 mois.

4. Utilisation selon l'une quelconque des revendications 1 à 3, dans laquelle l'antigène de rappel est administré au moins 2 fois.

5. Utilisation selon l'une quelconque des revendications 1 à 4, dans laquelle ladite molécule d'ADN est contenue dans un vecteur plasmidique sous le contrôle d'un promoteur hétérologue pour l'expression de la glycoprotéine de l'enveloppe chez l'hôte.

6. Utilisation selon la revendication 5, dans laquelle le promoteur hétérologue est le promoteur du cytomégalovirus.

7. Utilisation selon l'une quelconque des revendications 1 à 6, dans laquelle le vecteur est pCMV3Bx08 représenté dans la figure 2.

8. Utilisation selon l'une quelconque des revendications 1 à 7, dans laquelle l'antigène de primo-immunisation est administré au moins deux fois, et dans laquelle une période de repos pour que l'expansion clonale des lymphocytes B précurseurs spécifiques de l'antigène de primo-immunisation puisse s'effectuer chez l'hôte est respectée après chaque administration de ladite administration de primo-immunisation.

9. Utilisation selon l'une quelconque des revendications 1 à 8, dans laquelle le vecteur avipoxvirus atténué contient un génome de VIH modifié déficitaire en longues répétitions terminales, au moins le gène env étant celui de l'isolat primaire de BX08.

10. Utilisation selon la revendication 9, dans laquelle le virus ALVAC atténué est le vCP 1579.
